# FASCICULE DE BREVET EUROPEEN

(11) **EP 1 476 467 B1**
(45) Date de publication et mention de la délivrance du brevet: **07.06.2006**
(21) Numéro de dépôt: 03739532.4
(22) Date de dépôt: 13.02.2003
(51) Int. Cl.: C07K 16/06, C07K 16/08, C07K 16/10, A61K 39/42, G01N 33/68, A61K 47/48, A61K 51/10, G01N 33/567, G01N 33/569, A61P 31/00, A61P 31/18

(54) **IMMUNOGLOBULINE IGG3 MARQUEUR DE PROTECTION CONTRE LES MALADIES VIRALES INFECTIEUSES ET SES UTILISATIONS**
IGG3 IMMUNOGLOBLIN ALS SCHUTZSMARKER GEGEN VIREN INFEKTIÖSEN KRANKHEITEN UND SEINE VERWENDUNGEN
IMMUNOGLOBULIN IGG3 AS A MARKER FOR PROTECTING AGAINST INFECTIOUS VIRAL DISEASES, AND THE USES OF THE SAME

(30) Priorité: 15.02.2002 FR 0201960
(43) Date de publication de la demande: 17.11.2004
(73) Titulaire: URRMA R & D, 13400 Aubagne (FR); URRMA BIOPHARMA, Montréal, Quebec H3A 2L1 (CA)
(72) Inventeur: CHERMANN, Jean-Claude, F-13260 Cassis (FR); HASLIN, Camille, F-13009 Marseille (FR); DE OLIVEIRA Ricardo, AP-42-04532-03 S o Paulo, SP (BR)
(74) Mandataire: Ahner, Francis
(86) Numéro de dépôt international: PCT/FR2003/000463
(87) Numéro de publication internationale: WO 2003/068820

(56) Documents cités:
- US-A- 6 113 902
- GALEA P ET AL: "Identification of a biological marker of resistance to AIDS progression." CELLULAR PHARMACOLOGY, vol. 3, no. 5, 1996, pages 311-316, XP008010211 ISSN: 1351-3214
- SCHARF O ET AL: "Immunoglobulin G3 from polyclonal human immunodeficiency virus (HIV) immune globulin is more potent than other subclasses in neutralizing HIV type 1." JOURNAL OF VIROLOGY. UNITED STATES JUL 2001, vol. 75, no. 14, juillet 2001 (2001-07), pages 6558-6565, XP002219983 ISSN: 0022-538X
- KUNERT RENATE ET AL: "Stable recombinant expression of the anti HIV-1 monoclonal antibody 2F5 after IgG3/IgG1 subclass switch in CHO cells." BIOTECHNOLOGY AND BIOENGINEERING, vol. 67, no. 1, 5 janvier 2000 (2000-01-05), pages 97-103, XP002219984 ISSN: 0006-3592
- RAUX M ET AL: "IgG subclass distribution in serum and various mucosal fluids of HIV type 1-infected subjects." AIDS RESEARCH AND HUMAN RETROVIRUSES, vol. 16, no. 6, 10 avril 2000 (2000-04-10), pages 583-594, XP002219985 ISSN: 0889-2229
- HASLIN CAMILLE ET AL: "Anti-R7V antibodies as therapeutics for HIV-infected patients in failure of HAART." CURRENT OPINION IN BIOTECHNOLOGY, vol. 13, no. 6, 20 décembre 2002 (2002-12-20), pages 621-624, XP001161219 ISSN: 0958-1669

## Description

La présente invention se rapporte au domaine de la biologie et concerne plus particulièrement un nouveau variant d'immunoglobuline IgG3 isolée et/ou purifiée, utile comme marqueur de protection de maladies virales infectieuses telles le SIDA, et comme outil de diagnostic ou comme médicament préventif et curatif .

Le SIDA est un problème de santé publique extrêmement grave et préoccupant pour beaucoup de pays dans le monde. Ainsi aux USA par exemple, le nombre de cas de SIDA déclarés dépasse les 100 000 et le nombre de personnes infectées a été estimé à plus de un million. La propagation de la maladie est accentuée du fait du nombre de porteurs chroniques du virus responsable du SIDA qui restent asymptomatiques pendant de nombreuses années, sinon leur vie entière, et sont donc des sources de contamination non identifiées. Cette maladie, transmissible par voies sexuelle et sanguine affecte le système immunitaire de l'hôte, favorisant ainsi l'apparition d'infections opportunistes ou de pathologies contre lesquelles un système immunitaire sain aurait protégé l'hôte. Lorsque le SIDA est déclaré, la mort survient généralement deux à trois ans après le diagnostic suite à un effondrement des défenses immunitaires du patient et à de multiples infections opportunistes. Il est très difficile de classer les virus du SIDA vu l'extrême variabilité génétique et antigénique dont ils font preuve ; on admet classiquement qu'il existe deux types de virus responsables du SIDA humains : HIV-1 et HIV-2 (pour human immunodeficiency virus). Pour de multiples raisons, le mécanisme de réplication de HIV pose de nombreux problèmes pour l'obtention d'une thérapie efficace. En effet, l'ADN proviral de par son intégration dans le génome cellulaire se comporte comme un élément génétique de l'hôte. D'autre part, le virus HIV est disséminé à travers tout le corps dans les lymphocytes T, les monocytes, les macrophages ainsi que dans le système nerveux central. Enfin, le virus HIV possède une variabilité antigénique extrèmement importante. Les diverses thérapies curatives employées actuellement en clinique consistent pour l'essentiel soit à bloquer l'activité de la reverse transcriptase, soit à inhiber l'activité d'enzymes virales indispensables pour l'infection ou la réplication (protéases, intégrases). L'efficacité de ces thérapies reste limitée dans la mesure où l'absorption de ces antiviraux entraîne des effets secondaires. De plus, compte tenu du fort taux de mutation du virus HIV, celui-ci devient rapidement résistant aux drogues, tels l'AZT et aux autres analogues nucléotidiques durant la thérapie. L'émergence de résistants rend nécessaire l'augmentation des doses thérapeutiques administrées aux patients. L'échec des thérapies curatives actuelles nécessite donc le développement de nouvelles stratégies thérapeutiques pour combattre l'infection rétrovirale.

Dans leur recherche de développement de nouvelles stratégies curatives, les inventeurs ont été amenés à étudier les populations séropositives infectées par le HIV et non-progresseurs c'est-à-dire ne développant pas de SIDA. Ils ont ainsi pu mettre en évidence pour la première fois que chez ces patients séropositifs infectés par le HIV et qualifiés de non-progresseurs, il existe une variante particulière d'IgG3. Les inventeurs ont mis en évidence que cette variante d'IgG3 diffère par sa structure primaire de .poids moléculaire plus faible, sa demi-vie plus longue ainsi que par sa concentration sérique plus élevée que les IgG3 classiques. L'immunoglobuline IgG3 mise en évidence par les inventeurs, isolée et purifiée, apparaît comme un marqueur de protection pour le SIDA ainsi que comme un agent neutralisant de l'agent causal de cette maladie.

Les immunoglobulines IgG humaines se répartissent en quatre sous-classes (IgG1, IgG2, IgG3, IgG4) qui diffèrent par des différences mineures dans la structure primaire de leur chaîne lourde. Les principales différences concernent la région charnière et le nombre de ponts disulfure inter-chaînes. Ainsi la région charnière de l'IgG3 est très longue ce qui rend compte de son poids moléculaire plus élevé (170 kDa) et de certaines propriétés biologiques telles que la demi-vie (en jours) qui est beaucoup plus courte pour les immunoglobulines IgG3 (7 jours) que pour les autres sous-classes d'IgG qui ont une demi-vie de l'ordre de 20 jours. Les IgG3 sont capables de se lier sélectivement à certains récepteurs au fragment Fc des immunoglobulines tel que RFcγI, RFcγIIa, RFcγIIIa. Les immunoglobulines dont la fonction première est de lier l'antigène pour le neutraliser, ont également comme rôle d'activer des fonctions effectrices secondaires notamment par la voie du complément. Le système du complément qui est un groupe complexe de protéines sériques intervenant dans les réactions inflammatoires est un des mécanismes effecteurs le plus important pour les IgG1, IgG3 et les IgGM humaines. Après avoir lié l'antigène, les IgG1, les IgG3 et les IgGM peuvent en effet activer la cascade enzymatique de la voie classique du complément appelée C1q, les IgG2 quant à elles étant peu efficaces, les IgG4 en étant incapables. La liaison de C1q à l'immunoglobuline est la première étape dans la cascade du complément qui conduit à la lyse cellulaire. Ce mécanisme est particulièrement important pour combattre les maladies infectieuses et virales telles le SIDA par exemple et joue un rôle déterminant dans la destruction des cellules infectées.

Les travaux réalisés par les inventeurs permettent donc de répondre à un besoin urgent, à savoir la nécessité de développer des nouveaux médicaments ou compositions préventives et/ou curatives destinées à neutraliser le virus du SIDA, ainsi qu'à développer de nouveaux tests de diagnostic permettant d'adapter la thérapie en fonction du sérotype du patient.

Le brevet américain US 6,113,902 délivré le 5 septembre 2000 (CHERMANN et al.) décrit l'utilisation de l'épitope R7V pour détecter des anticorps dans le sérum de patients et pour démontrer la présence d'anticorps protecteurs dans le sérum des patients séropositifs qui n'ont pas développé le SIDA.

Le document GALEA et al. (Cellular Pharmacology, vol.3, no.5, 1996, p.311-316) divulgue l'existence d'anticorps dirigés contre l'épitope R7V et le fait que leur présence soit associée à une activité neutralisante de certaines souches de HIV in vitro et à une forme asymptomatique de la maladie.

L'IgG3 selon la présente invention diffère de ces antisérums par le fait qu'il s'agit du composé purifié et actif du mélange constitué par l'antisérum.

Le document SCHARF et al. (J. Vir. July 2001, vol. 75, no. 14, p.6558-6565) divulgue l'existence d'immunoglobulines de type IgG3 dirigées contre le virus HIV et l'utilisation éventuelle de leur activité neutralisante pour une immunisation passive.

L'IgG3 selon la présente invention possède des caractéristiques différentes d'une IgG3 standard, à savoir une durée de vie plus longue, une chaîne lourde de poids moléculaire plus faible et une concentration sérique plus élevée.

La présente invention a pour objet une immunoglobuline humaine de classe IgG3 purifiée et/ou isolée, ayant les caractéristiques suivantes (i) une durée de vie dans le sérum du patient supérieure à la durée de vie des IgG3 normalement présente dans le sérum humain et notamment d'au moins 15 jours de préférence d'au moins un mois , (ii) une chaîne lourde dont le poids moléculaire, déterminé par mobilité électrophorétique, est inférieur au poids moléculaire des IgG3 normalement présentes dans le sérum humain (60kDa), la dite chaîne lourde ayant un poids moléculaire d'environ 50 kDa et comprenant le site de fixation du complément. Cette réduction du poids moléculaire apparant déterminée par électrophorèse reflète soit une diminution de la longueur de la structure primaire de l'IgG3, soit une conformation tridimensionnelle différente, soit une diminution des modifications post-traductionnelles telles par exemple la glycosylation de la chaîne lourde.

L'immunoglobuline IgG3 variante selon l'invention dans le sérum se caractérise enfin par une concentration sérique supérieure à la concentration d'IgG3 d'un sérum normal d'au moins une fois, d'au moins deux fois, en général d'au moins trois fois, ce qui représente une concentration sérique de cette nouvelle IgG3 variante d'environ 1 g/l.

Selon un mode de réalisation de l'invention, l'immunoglobuline variante IgG3 est isolée et/ou purifiée à partir des patients séropositifs pour le HIV ; ces patients étant non-progresseurs, c'est-à-dire des personnes infectées par la VIH depuis plusieurs années sans perte de défenses immunitaires donc asymptomatiques. De manière plus préférée, l'immunoglobuline IgG3 selon l'invention lie sélectivement l'épitope R7V de la protéine gp160 du virus HTV-1, le dit épitope comprenant la séquence Arg-Thr-Pro-Lys-Ile-Gln-Val.

Dans la présente description, les termes immunoglobulines et anticorps seront employés indifféremment. Par épitope, on entendra désigner tout déterminant de la protéine responsable de l'interaction spécifique avec l'anticorps ; les épitopes consistent habituellement en des groupes de molécules présentant des surfaces chimiquement actives telles que des acides aminés ou des chaînes latérales de sucres et ayant une structure tridimentionnelle spécifique et/ou une charge spécifique caractéristique.

Les procédés d'isolement et/ou de purification des immunoglobulines, les méthodes pour déterminer la longueur de la chaîne lourde des immunoglobulines ainsi que les procédés de détermination de la durée de vie des IgG3 dans le sérum sont connues de l'homme du métier travaillant dans le domaine de l'immunologie. A titre d'illustration, différents procédés et protocoles pouvant être mis en oeuvre sont décrits dans Current Protocols in Immunology" mise à jour annuellement (4volumes) édités par le "National Institutes of Health" par John E. Coligan, Ada M. Kruisbeek, David H. Margulies, Ethan M. Shevach, Warren Strober- Wiley Interscience.

A titre illustratif, la purification des IgG3 de la présente invention est réalisée en utilisant des colonnes d'immuno-affinité Hitrap^{™} Protein G HP (réf. 17-0404-01) et Hitrap^{™} Protein A HP (réf. 17-0402-01) de chez Amersham-Pharmacia. Après le passage du sérum/plasma sur la colonne Hitrap^{™} protein A HP qui fixe uniquement les IgG1, IgG2 et IgG4, le sérum/plasma épuisé en IgGI, IgG2 et IgG4 est passé sur la colonne Hitrap^{™} Protein G HP pour purifier les IgG3

La détermination du poids moléculaire des chaînes lourdes et légères des IgG est réalisée par exemple par électrophorèse sur un gel de polyacrylamide 12% (Ready Gel Tris-HCl glycine BIORAD réf. 161-0901). Brièvement, les IgG3 purifiées sur colonne d'immunoaffinité sont réduits par chauffage 3 minutes à 95°C en présence de tampon réducteur (Laemmli sample Buffer BIORAD réf. 161-0737 +β mercaptoéthanol) puis chargées sur le gel. La migration s'effectue en 40 minutes à 200 V, 35 mA. Les gels sont colorés par une solution commerciale (Bio-Safe Coomassie BIORAD, réf. 161-0786).

Enfin, la détermination de la durée de vie des IgG3 variantes est réalisée par un test ELISA réalisé chaque mois ou chaque trimestre dans le sérum ou plasma de patients non progresseurs permettant de confirmer la présence d'IgG3.

Par isolement de l'immunoglobuline IgG3 variant selon l'invention on couvre également la préparation d'anticorps monoclonal de type IgG3 variant. Pour la préparation d'anticorps monoclonaux ou leurs fragments, on pourra se référer aux techniques qui sont en particulier décrites dans le manuel « Antibodies » (Harlow et al., 1988) ou à la technique de préparation à partir d'hybridomes décrite par Kohler et Milstein en 1975. Les anticorps monoclonaux selon l'invention peuvent être obtenus par exemple à partir de cellule d'un animal immunisé contre la protéine gp160, ou un de ses fragments, et comportant l'épitope R7V. La purification de l'anticorps selon l'invention pourra par exemple être réalisée sur une colonne d'affinité sur laquelle a préalablement été immobilisée l'antigène ou l'épitope cible, par exemple l'épitope R7V de la protéine gp160 du HTV.

Le concept inventif à la base de l'invention ne se réduit pas aux seules immunoglobulines IgG3 purifiées et/ou isolées de patient affectés du SIDA . En effet, le type d'immunoglobulines lgG3 variant mises en évidence par les inventeurs sont exprimées également dans le sérum de patients affectés d'autres pathologies impliquant une infection virale. Parmi ces pathologies impliquant une infection par les virus, on peut citer à titre d'exemples non exhaustifs les pathologies associées aux virus de la leucémie des cellules T humaines (HTLV), le cytomégalovirus (CMV), les virus de l'herpès (HSV-1, HSV-2), le virus d'Epstein-Barr (EBV), les virus de l'hépatite (HBV, HCV). L'invention concerne également les immunoglobulines lgG3 variant isolées et/ou purifiées à partir d'un patient affecté de maladie infectieuse à évolution variable comme les maladies neurologiques telles la sclérose en plaque, les maladies cutanées telles le psoriasis, les maladies auto-immunes telles le lupus érythémateux disséminé, les cancers d'origine rétroviral tels le cancer du sein inflammatoire familial.

L'homme du métier a, à sa disposition, les outils de biologie moléculaire et cellulaire pour réaliser le clonage, le séquençage, et l'expression par voie recombinante des immunoglobulines IgG3 variantes (Sambrook *et al*. 1989 ; Coligan et al., Current Protocols in Immunology voir page 4). Ces IgG3 recombinantes, également dans la portée de la présente invention, pourront ainsi être produites *in vitro* et facultativement modifiées par la technologies de l'ADN recombinant ou de la chimie pour leur conférer des propriétés particulières.

Suivant les applications diagnostic désirées, l'anticorps selon l'invention peut être immobilisé sur un support. L'immobilisation ou couplage peut être réalisée sur de nombreux supports connus de l'homme de l'art. Cette immobilisation ou couplage est réalisée de préférence sur un support solide directement ou indirectement par l'intermédiaire d'un bras d'espacement. Les supports solides peuvent notamment inclure le verre, le polystyrène, le polypropylène, le polyéthylène, le dextran, le nylon, ou des celluloses naturelles ou modifiées. Ces supports peuvent être soit solubles ou insolubles.

Pour d'autres applications, l'anticorps selon l'invention peut être marqué directement ou indirectement par un marqueur de façon à obtenir un conjugué permettant de générer un signal détectable et/ou quantifiable utilisable pour le diagnostic *in vivo* ou *in vitro*. Le kit de diagnostic correspondant comprenant l'anticorps marqué est également un des objets de la présente invention. Le marqueur peut être sélectionné parmi les enzymes, les colorants, les haptènes, les agents luminescents tels que les agents radioluminescents, chémiluminescents, bioluminescents, fluorescents, phosphorescents, les ligands tels que la biotine, l'avidine, la streptavidine, la digoxygénine, les isotopes radioactifs. Ainsi, l'immunoglobuline selon l'invention est conjuguée par exemple avec des enzymes telles que la péroxydase, la phosphatase alkaline, la β-D-galactosidase, la glucose oxydase, la glucose amylase, l'anhydrase carbonique, l'acétyl-cholinestérase, le lysozyme, la malate déhydrogénase ou la glucose-6 phosphate déhydrogénase ou par une molécule comme la biotine, la digoxigénine ou la 5-bromo-désoxyuridine. Des marqueurs fluorescents peuvent être également conjugués à l'immunoglobuline selon l'invention et incluent par exemple la fluorescéine et ses dérivés, la rhodamine, le rouge Texas, le dansyl, l'umbelliférone, et les protéines autofluorescentes telles la GFP (GFP pour « Green Fluorescent Protein ») etc.. D'autres conjugués peuvent inclure également des marqueurs chemiluminescents tels que le luminol et les dioxétanes ou des marqueurs bioluminescents tels que la luciférase et la luciférine.

Parmi les isotopes radioactifs pouvant être fixés sur l'immunoglobuline selon l'invention, on préfère également les marqueurs radioactifs tels que ¹⁴C, ³⁶Cl, ⁵⁷Co, ⁵⁸Co, ⁵¹Cr, ¹⁵²Eu, ⁵⁹Fe, ³H, ¹²⁵I, ¹³¹I, ³²P, ³⁵S, ⁷⁵Se et ^{99m}Tc qui peuvent être détectés par des moyens connus tels que le compteur gamma ou à scintillations, par autoradiographie, etc.. La présente invention comprend également les conjugués dont le marqueur détectable est choisi parmi les marqueurs pouvant être utilisés dans l'application imagerie *in vivo*. Des exemples de tels marqueurs selon l'invention sont ⁷²As, ⁶⁷Cu, ⁶⁷Ga, ⁶⁸Ga, ¹²³I, ¹²⁵I, ¹³¹I, ¹¹¹In, ⁹⁷Ru, ^{99m}Tc, ²⁰¹Tl et ⁸⁹Zr. L'invention inclut également les isotopes paramagnétiques utilisés dans l'imagerie par résonance magnétique (IRM) et qui incluent notamment ⁵²Cr, ¹⁶²Dy, ⁵⁶Fe, ¹⁵⁷Gd et ⁵⁵Mn. Le terme « imagerie in vivo » doit être entendu dans la présente description comme toute méthode permettant la détection d'un anticorps marqué selon la présente invention qui se fixe spécifiquement sur l'épitope de la protéine d'intérêt dans le corps du patient. Le patient sera de préférence un homme susceptible de présenter des cellules infectées par un virus ou atteint d'une maladie infectieuse à évolution variable exprimant de manière anormale la protéine d'intérêt.

De tels conjugués peuvent être préparés par des méthodes connues de l'homme de l'art. Ils peuvent être couplés aux marqueurs directement ou par l'intermédiaire d'un groupe fonctionnel intemiédiaire, d'un groupe espaceur ou d'un groupe de liaisons tel qu'un polyaldéhyde, comme le glutaraldéhyde, l'acide éthylènediaminetétraacétique (EDTA), l'acide diéthylènetriaminepentaacétique (DPTA), ou en présence d'agents de couplage tels que le périodate, la benzoquinone etc.. Les conjugués comportant des marqueurs de type fluorescéine peuvent être préparés par réaction avec un iso-thiocyanate.

C'est également un des objet de la présente invention de fournir un procédé de diagnostic et/ou de dosage *in vitro* d'une pathologie sélectionnée parmi les infections virales et les maladies infectieuses à évolution variable, comprenant les étapes de (i) mise en contact d'un échantillon de fluide corporel et/ou de tissu corporel provenant d'un patient chez lequel on suspecte la présence de la dite pathologie avec une immunoglobuline selon l'invention; et (ii) de détection de la présence d'un complexe immun entre la dite immunoglobuline et un épitope spécifique de la dite pathologie. Par fluide corporel, ou fluide biologique, on entend désigner des fluides tels le sérum, le sang total, l'urine, le sperme, les cellules, un échantillon de tissu ou des biopsies d'origine humaine. Les méthodes de détection du complexe immun sont multiples et connues de l'homme de l'art ; elles dépendent de la nature du diagnostic et du dosage à effectuer. Il peut s'agir de test ELISA, RLA, d'une méthode sandwich, d'une immuno-précipitation, d'un test d'agglutination, d'un test par compétition; ou tout test connu de l'homme de l'art dépendant de la formation d'un complexe immun anticorps-antigène. A titre d'exemple, une méthode préférée met en jeu des processus immuno-enzymatiques selon la technique ELISA, par immunofluorescence, ou radio-immunologique (RIA) ou équivalent. Toutes ces méthodes reposent de préférence sur la fixation des immunoglobulines en cause sur des peptides antigéniques puis par la mise en évidence de cette fixation.

Plus particulièrement, l'invention couvre également un procédé de diagnostic et/ou de dosage *in vitro* du syndrome d'immunodéficience humaine (SIDA) comprenant les étapes de (i) mise en contact d'un échantillon de fluide corporel et/ou de tissu corporel provenant d'un patient chez lequel on suspecte la présence du HIV avec une immunoglobuline selon l'invention et, (ii) de détection de la présence d'un complexe immun entre la dite immunoglobuline et la protéine gp160 du HIV, et notamment l'épitope R7V de cette protéine contenu dans le dit échantillon.

L'invention concerne également un procédé *in vitro* ou *in vivo* de traitement de fluide biologique d'un patient affecté d'une pathologie sélectionnée panni les infections virales, notamment le HIV, et les maladies infectieuses à évolution variable, le dit procédé comprenant l'étape de mettre en contact une quantité efficace d'immunoglobuline, selon l'invention, avec le dit fluide biologique, de sorte à neutraliser un épitope spécifique de la pathologie dans le dit fluide. Le procédé comprend en outre éventuellement l'étape additionnelle d'éliminer du mélange récationnel le complexe immun ainsi formé. Dans le cadre d'un procédé *in vitro* de traitement du fluide biologique, le procédé peut comprendre en outre l'étape additionnelle de réinjectcr au patient tout ou partie du fluide biologique ainsi traité. Par neutralisation du virus HIV par exemple, on entendra désigner tout mécanisme ayant pour effet *in vivo* de détruire et/ou d'empêcher la propagation des virus. L'immunoglobuline variant d'IgG3 selon l'invention apparaît compte tenu de son sous type G3 et de sa stabilité, constituer un excellent moyen pour neutraliser tout fluide corporel destiné à être réinoculé ou réintroduit chez un individu, comme le sang d'un homme HTV séropositif pour la transfusion sanguine d'un individu, un tissu d'un homme HIV séropositif dans le cas d'une greffe tissulaire, ou le sperme d'un homme HIV séropositif pour l'insémination d'une femme séronégative. En effet, le sous-type IgG3 fixant le complément, les immunoglobulines selon l'invention induisent la lyse des cellules infectées.

Selon un mode particulier de réalisation, l'invention fournit un procédé *in vitro* de neutralisation de cellules infectées par le HIV dans une échantillon biologique de fluide corporel et/ou de tissu corporel provenant d'un patient séropositif. Ce procédé comprend les étapes suivantes de :
(i) combiner de manière simultanée, séparée ou étalée dans le temps l'immunoglobuline selon l'invention avec l'échantillon biologique contenant les cellules infectées par le HIV présentant la protéine gp160 à leur surface et avec de la protéine G attachée à des particules magnétiques ;
(ii) Incuber le mélange obtenu en (i) dans des conditions permettant la fixation de la dite immunoglobuline sur la protéine gp160, et de préférence sur l'épitope R7V, pour former un complexe, le dit complexe comprenant le dit anticorps lié à une cellule infectée par le HIV fixé sur cette particule magnétique ;
(iii) Déplacer cette particule magnétique vers un endroit prédéterminé du récipient contenant ce mélange réactionnel, tel que le déplacement est accompli par un champ magnétique agissant sur la dite particule magnétique.

L'invention couvre également un procédé *in vitro* de diagnostic de patient non-progresseur affecté d'une pathologie sélectionnée parmi les infections virales, notamment le SIDA et les maladies infectieuses à évolution variable caractérisé en ce que l'on détecte de préférence par un test immunologique la présence d'immunoglobuline IgG3 ayant les caractéristiques suivantes (i) une durée de vie dans le sérum du patient supérieure à celle des IgG3 normalement présente, et notamment d'au moins 15 jours, de préférence d'au moins un mois ; (ii) une chaîne lourde dont le poids moléculaire, déterminé par mobilité électrophorétique, est inférieur au poids moléculaire des IgG3 normalement présentes dans le sérum humain (60kDa), la dite chaîne lourde ayant un poids moléculaire d'environ 50 kDa et comprenant le site de fixation du complément., et (iii) facultativement ayant une concentration sérique supérieure d'au moins une fois, d'au moins deux fois, d'au moins trois fois (soit 1g/l) à la concentration d'lgG3 d'un sérum nornaal. Le test de diagnostic utilisé est de préférence choisi parmi un test ELISA, RIA, une méthode sandwich, une immuno-précipitation, un test d'agglutination. Ce diagnostic de détection de la variante IgG3 selon l'invention présente un intérêt considérable. En effet, les patients séropositifs dans le cas du HIV et porteurs de cette variante d'IgG3 sont non-progresseurs. Dans ce cas le diagnostic est très favorable et il est possible d'éviter des traitements thérapeutiques lourds. Ceci est particulièrement vrai dans le cas d'une grossesse où la présence de ces anticorps chez la mère (H1V+) semblerait conduire à une non-infection du nouveau-né. La présente invention vise donc à utiliser l'immunoglobuline IgG3 variante selon l'invention comme un marqueur de protection dans les pathologies sélectionnées parmi les maladies virales, notamment le SIDA et les maladies infectieuses à évolution variable.

Plus généralement, la présente invention vise à couvrir l'utilisation d'une immunoglobuline selon l'invention pour la réalisation d'un test immunologique choisi parmi le test ELISA, le test RIA, la méthode sandwich, l'immuno-précipitation, le test d'acolutination.

La présente invention vise également à couvrir la mise en couvre de l'immunoglobuline selon l'invention à titre de médicament, et plus précisément comme anticorps thérapeutique ou comme agent de ciblage. Par anticorps thérapeutique, on entend désigner des immunoglobulines IgG3 ou un sérum, plasma le contenant, injectées par voie veineuse à des patients progresseurs et généralement en échec de thérapie classique.

L'immunoglobuline lgG3 variant est utile à la préparation de médicament destiné au traitement thérapeutique et/ou prophylactique d'une maladie virale, notamment le SIDA, mais également des maladies dont l'agent causal est un virus tel que listé précédemment. Lorsque la maladie est le SIDA, l'immunoglohuline IgG3 variant utilisée pour la préparation d'un médicament destiné à neutraliser chez un homme affecté du SIDA la protéine gp160 de HIV est de préférence dirigée contre l'épitope R7V de la protéine gp160 du HIV. L'immunoglobuline IgG3 variant est également utile à la préparation de médicament destiné au traitement thérapeutique et/ou prophylactique d'une maladie infectieuse à évolution variable.

L'immunoglobuline IgG3 selon l'invention, peut être mise en oeuvre comme médicament sous la forme d'un mélange avec au moins un agent anti-rétroviral choisi parmi le groupe composé des inhibiteurs de la reverse transcriptase et/ou des antiprotéases virales comme produit de combinaison pour une utilisation simultanée, séparée ou étalée dans le temps en thérapie antivirale. L'inhibiteur de la reverse transcriptase est de préférence choisi parmi le 3'-azido-3'déoxythymidine (AZT), le 2',3'-didéoxyinosine (ddI), le 2',3'-didéoxycytidine (ddC), le (-)2',3'-didéoxy-3'-théacytidine (3TC), le 2',3'-didéhydro-2',3'didéoxythymidine (d4T) et le (-)2'-déoxy-5-fluoro-3'-théacytidine (FTC), le TIBO, le HEPT, le TSAO, l'α-APA, la névirapine, le BAHP, l'acide phosphonoformique (PFA) ; l'antiprotéase virale est choisie parmi l'indinavir et le saquinavir.

Dans le cadre d'une mise en oeuvre de l'anticorps comme agent de ciblage, celui-ci est éventuellement modifié par les technologies de la chimie et/ou de l'ADN recombinant, pour modifier sa stabilité, son affinité, sa bio-disponibilité ou sa compatibilité etc... L'immunegiobuline selon l'invention comme agent de ciblage est conjuguée directement ou indirectement à au moins un agent sélectionné parmi le groupe des agents antiprolifératifs, antinéoplastiques ou cytotoxiques. Parmi les agents qui peuvent être conjugués aux anticorps selon l'invention sont inclus, outre les radio-isotopes et les marqueurs précédemment décrits notamment, les composés alkylants tels que la méchloréthamine, la triéthylène phosphoramide, la triaziquone, la camustine, la sémustine, le méthotrexate, la mercaptopurine, la cytarabine, le fluorouracile, les antibiotiques tels que l'actiriomycine, les hormones ou les antagonistes d'hormones tels que les corticostéroïdes, comme la prednisone ou les progestines, les toxines bactériennes ou virales. Dans un mode particuler de réalisation, l'agent antiprolifératif et/ou antinéoplasique et/ou cytotoxique non isotopique est une molécule d'acide nucléique telle par exemple de l'ADN simple brin, de l'ADN double brin, de l'ARN simple brin, notamment un ARN antisens, de l'ARN double brin, un hybride ARN/ADN. Cet acide nucléique code facultativement pour un produit protéique d'intérêt.

Il entre également dans la portée de l'invention de fournir une vaccin ou une composition pharmaceutique pour le traitement ou la prévention d'une pathologie sélectionnée parmi les infections virales et les maladies infectieuses à évolution variable comprenant une immunoglobuline anticorps selon l'invention en association avec un transporteur, un excipient ou un diluant pharmaceutiquement acceptable. Les excipients pharmaceutiquement acceptables incluent notamment l'eau, les solutions salines, les tampons ou tout autre composé décrit par exemple dans l'Index de Merck.

Les compositions ou vaccin selon l'invention peuvent comporter également des composants augmentant l'immunogénicité, notamment des adjuvants d'immunité spécifiques ou non tels que l'adjuvant de Freund, des polysaccharides ou des composé équivalents. Il s'agit là de composés connus par l'homme du métier dans le domaine de la vaccination. Les compositions peuvent être sous une forme quelconque compatible avec la voie d'administration choisie. L'administration de ces composés au patient peut être locale ou systémique et accomplie par voie intraveineuse, intra-artérielle, intra-musculaire, intra-péritonéale, par l'intermédiaire du liquide spinal, par voie intradennique, par voie orale, nasale, anale.. Toutefois Jes compositions selon la présente invention pourront être utilisées par d'autres voies, notamment par voie aérosol, pour induire une protection des muqueuses.

La quantité d'immunoglobulines IgG3 variant, comprise dans les compositions pharmaceutiques selon la présente invention et nécessaire pour une thérapie efficace dépendra de différents facteurs tels que le mode d'administration, la partie du corps ciblée, l'état physiologique et l'âge du patient, des éventuels effets secondaires, de contre-indications s'il en existe, de thérapies concomittantes ou d'autres variables qu'un homme de l'art saura ajuster. Le dosage pour de telles préventions ou traitements thérapeutiques devra être réalisé de manière à optimiser sa sécurité et son efficacité. En général, les dosages utilisés *in vitro* peuvent fournir une indication pour les quantités utilisées pour l'administration d'anticorps *in situ* et ainsi des modèles animaux peuvent être utilisés pour déterminer les quantités efficaces d'immunoglobulines selon l'invention pour le traitement de pathologie particulière, notamment du SIDA.

Les anticorps monoclonaux selon l'invention constituent également un moyen d'analyse immunocytochimique ou immunohistochimique de l'expression de la protéine d'intérêt sur des gels d'électrophorèse ou des membranes de tranferts, ou des coupes de tissus spécifiques, par exemple par immunofluorescence, par marquage enzymatique, radioactif ou à l'or. Ils permettent notamment de mettre en évidence et de quantifier la présence spécifique normale ou anormale de la protéine d'intérêt dans les tissus ou prélèvements biologiques, ce qui les rend utiles pour l'identification et la localisation de l'expression de la protéine d'intérêt, pour le diagnostic de pathologies liées à la présence anormale de la protéine d'intérêt, tel par exemple la gp160, mais également pour le suivi de l'évolution de méthodes de prévention ou de traitement de pathologie nécessitant ladite détection ou ledit dosage. Plus généralement, les anticorps selon l'invention peuvent être avantageusement mis en oeuvre dans toute situation où l'expression d'une protéine d'intérêt, la protéine gp160 contenant l'épitope R7V par exemple, doit être observée de manière qualitative et/ou quantitative.

D'autres caractéristiques et avantages de l'invention apparaissent dans la suite de la description avec les exemples et les figures dont les légendes sont représentées ci-après.

### FIGURES

**FIGURE 1**
   1a : Comparaison d'une IgG₃ d'un sérum normal (SN1) avec celle d'un patient HIV positif non-progresseur (ARG).
   1b : Confirmation du poids moléculaire plus faible de la chaîne lourde avec un autre patients (ETC).
   1c : Autres patients (THO, GEM)
   1d : Autre patient (VAL) et autre séronégatif (SN2).
   1e : Autre patient (BOI)
**FIGURE 2.**
   Protocole d'immuno-précipitation du rétrovirus VIH par l'IgG3 variant à titre d'exemple.
**FIGURE 3.**
   Immuno-précipitation du virus par les IgG₃ de trois patients différents.
**FIGURE 4.**
   Immuno-précipitation de quantités variables de virus avec une quantité fixe d'anticorps IgG3.
**FIGURE 5.**
   Immuno-précipitation de souches différentes et éloignées entre elles par une quantité fixe d'IgG3.
**FIGURE 6.**
   Schéma résumant le protocole de neutralisation des virus.

### EXEMPLES

### EXEMPLE 1 : Mise en évidence de variantes d'IgG3 chez certains sujets infectés par le VIH qualifiés de non progresseurs.

Les immunoglobulines anticorps se divisent en quatre sous-classes et généralement les anticorps induits après immunisation appartiennent aux IgG₁, qui ont une demi-vie très longue.

Les inventeurs décrivent ici chez des patients infectés par le VIH, la présence d'anticorps protecteurs associés aux IgG₃, alors que les anticorps signant l'infection sont toujours des IgG₁. Une variante d'IgG₃ existe chez certains sujets infectés par le VIH qualifiés de non progresseurs.

### 1.1. La variante IgG3 a une durée de vie de plusieurs mois

Cette variante diffère des IgG₃ classiques par sa demi-vie ; elle est de sept jours pour les IgG₃ classiques et de plusieurs mois pour la variante, comme le montre 1'.étude sur les suivis séquentiels de patients, les sérums étant prélevés à plusieurs mois d'intervalle (tableau 1).

Sur le tableau 1, on voit les résultats d'un test ELISA pour la recherche des anticorps protecteurs dirigés contre un épitope associé au HIV, appelé R7V. Ces anticorps sont révélés par des anticorps seconds distinguant les différentes sous-classes des immunoglobulines humaines.

Les anticorps protecteurs anti-R7V sont toujours des IgG₃ ou / et IgG₄, mais jamais des IgG₁ ou IgG₂, et leur titre élevé se maintient plusieurs mois.

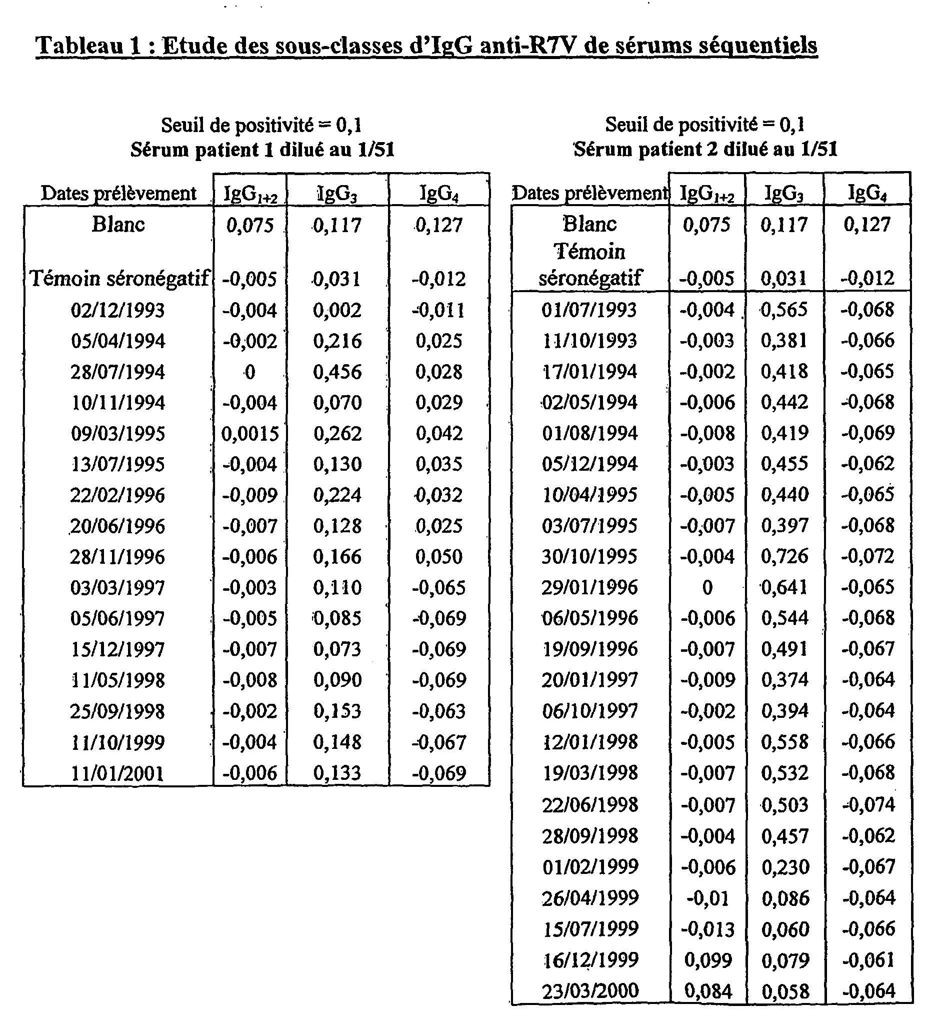

### 1.2. La variante IgG3 a une chaîne lourde d'environ 50 kDa

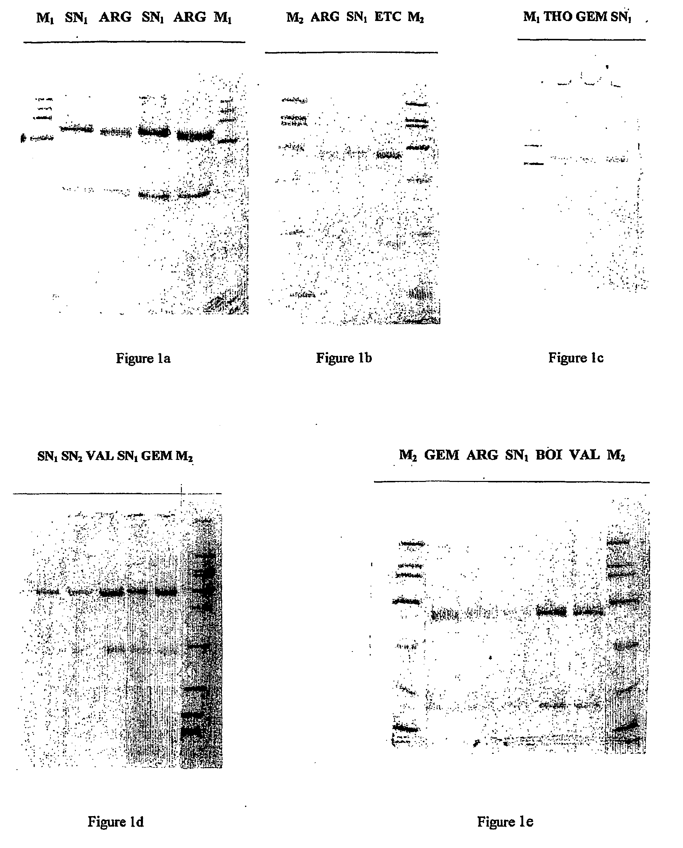

Les analyses électrophorétiques des figures 1 montrent que la variante de l'IgG₃ présente chez les patients non-progresseurs infectés par le VIH a une chaîne lourde à mobilité électrophorétique plus grande, ce qui signifie que son poids moléculaire est plus faible que celui d'une IgG₃ classique purifiée chez un sujet normal HIV négatif.

Les figures **1a** à **1e** montrent la migration des IgG₃ purifiées soit à partir de sujets normaux HIV négatifs, soit à partir de patients HIV positifs sur un gel de polyacrylamide 12%. Les chaînes lourdes ont un poids moléculaire aux alentours de 50 kda, alors que les chaînes légères ont un poids moléculaire de 25 kda. (Marqueurs de poids moléculaire : M₁ :250 ; 150 ; 100 ; 75 ; 50 ; 37 ; 25 ;15 ; 10 kda / M₂ : 200 ; 116 ; 97 ; 66 ; 45 ; 31 ; 21,5 ;14,4 ; 6,5 kda)

La variante de l'IgG₃ est reconnue par des anticorps dirigés soit contre la partie Fab des IgG₃ soit des anticorps dirigés contre la partie charnière. La variation devrait donc porter sur le fragment Fc, probablement au niveau de la glycosylation.

### 1.3. La variante de l'IgG₃ est retrouvée chez la majorité des HIV séropositifs non-progresseurs (tableau 2).

**Tableau 2 : Détermination de la sous-classe des IgG anti-R7V par ELISA**

| Seuil de positivité = 0,2. | | | | | |
|---|---|---|---|---|---|
| | | **IgG**_{**1**} | **IgG**_{**2**} | **IgG**_{**3**} | **IgG**_{**4**} |
| | Blanc | 0,058 | 0,056 | 0,085 | 0,081 |
| | Témoin séronégatif 1/50 | 0,059 | 0,060 | 0,113 | 0,091 |
| | Témoin positif | 0,057 | 0,057 | 0,854 | 0,084 |
| 1 | ALB HE 1/50 (07/01/94) | 0,072 | 0,063 | 0,080 | 0,073 |
| 2 | ANO MA 1/50 (13/11/00) | 0,061 | 0,057 | 0,136 | 0,108 |
| 3 | ARG CH 1/50 (30/11/00) | 0,058 | 0,055 | 0,481 | 0,071 |
| 4 | AUC LA 1/50 (15/11/99) | 0,065 | 0,061 | 0,112 | 0,138 |
| 5 | AUR PH 1/50 (03/01/94) | 0,069 | 0,064 | 0,343 | 0,685 |
| 6 | BEN MI 1/50 (14/02100) | 0,062 | 0,064 | 0,077 | 0,071 |
| 7 | BER YV 1/50 (13/03/00) | 0,105 | 0,073 | 0,109 | 0,307 |
| | BER YV 1/50 (25/11/99) | 0,102 | 0,072 | 0,107 | 0,339 |
| 8 | BITT MA 1/50 (27/05/99) | 0,060 | 0,054 | 0,384 | 0,091 |
| 9 | BOI CH 1/50 (27/11/00) | 0,060 | 0,055 | 0,236 | 0,092 |
| 10 | BOU NA 1/50 (23/12/99) | 0,070 | 0,067 | 0,119 | 0,081 |
| 11 | CAM GE 1/50 (27/11/00) | 0,063 | 0,072 | 0,079 | 0,077 |
| 12 | COU DI 1/50 (16/09/98) | 0,094 | 0,073 | 0,081 | 2,468 |
| | COU DI 1/50 (09/03/00) | 0,074 | 0,070 | 0,077 | 1,935 |
| | COU DI 1/50 (08/09/00) | 0,058 | 0,059 | 0,059 | 2,641 |
| 13 | CUC JE 1/50 (28/09/00) | 0,059 | 0,056 | 0,173 | 0,088 |
| 14 | DAN NO 1/50 (28/09/00) | 0,073 | 0,066 | 0,083 | 0,094 |
| 15 | DAR DO 1/50 (10/03/94) | 0,078 | 0,066 | 0,795 | 0,085 |
| 16 | DOM JO 1/50 (13/07/92) | 0,060 | 0,057 | 0,275 | 0,096 |
| 17 | ESM GU 1/50 (18/12/00) | 0,061 | 0,064 | 0,083 | 0,119 |
| 18 | ETC MA 1/50 (25/09/98) | 0,114 | 0,067 | 1,266 | 0,089 |
| | ETC MA 1/50 (13/06/00) | 0,057 | 0,057 | 0,854 | 0,084 |
| 19 | FER JE 1/50 (26/06/00) | 0,064 | 0,070 | 0,079 | 0,204 |
| 20 | FIN RO 1/50 (13/11/00) | 0,063 | 0,065 | 0,097 | 0,089 |
| 21 | FON RE 1/50 (29/06/00) | 0,083 | 0,053 | 0,095 | 0,070 |
| 22 | GEM SA 1/50 (20/10/98) | 0,093 | 0,071 | 0,090 | 0,086 |
| | GEM SA 1/50 (29/04/99) | 0,060 | 0,061 | 0,114 | 0,080 |
| | GEM SA 1/50 (07/06/00) | 0,078 | 0,050 | 0,079 | 0,068 |
| 23 | GOU JE 1/50 (13/06/00) | 0,069 | 0,052 | 0,098 | 0,072 |
| 24 | KAZ AL 1/50 (20/01/01) | 0,059 | 0,065 | 0,117 | 0,080 |
| 25 | KOH MI 1/50 (05/08/94) | 0,056 | 0,056 | 0,869 | 0,088 |
| 26 | MAN GU 1/50 (06/04/00) | 0,074 | 0,053 | 0,072 | 0,079 |
| 27 | MART DO 1/50 (03/05/00) | 0,082 | 0,064 | 0,311 | 0,077 |
| | MART DO 1/50 (09/05/94) | 0,082 | 0,067 | 0,329 | 0,079 |
| 28 | MART PA 1/50 (13/06/00) | 0,055 | 0,058 | 0,106 | 0,083 |
| 29 | MEC EV 1/50 (28/01/00) | 0,068 | 0,054 | 0,086 | 0,074 |
| 30 | MEN CH 1/50 (09/03/00) | 0,051 | 0,053 | 0,078 | 0,155 |
| 31 | MOR AN 1/50 (20/04/00) | 0,075 | 0,065 | 0,109 | 0,086 |
| 32 | PAT MA 1/50 (09/07/98) | 0,086 | 0,072 | 0,116 | 0,085 |
| | PAT MA 1/50 (07/07/00) | 0,054 | 0,053 | 0,104 | 0,070 |
| | PAT MA 1/50 (11/06191) | 0,082 | 0,067 | 0,152 | 0,067 |
| 33 | REF EL 1/50 (28/09/00) | 0,070 | 0,067 | 0,085 | 1,153 |
| 34 | RIG ST 1/50 (02/02/98) | 0,089 | 0,070 | 0,131 | 0,096 |
| 35 | RIO EM 1/50 (11/06192) | 0,071 | 0,067 | 0,115 | 0,073 |
| 36 | ROB IS 1/50 (26/06/00) | 0,054 | 0,056 | 0,095 | 0,068 |
| 37 | ROD CH 1/50 (12/04/99) | 0,057 | 0,055 | 0,155 | 0,068 |
| 38 | SAN FR 1/50 (19106/00) | 0,059 | 0,054 | 0,139 | 0,088 |
| 39 | SAU CH 1/50 (20/12/91) | 0,074 | 0,069 | 0,092 | 0,640 |
| | SAU CH 1/50 (27111/00) | 0,059 | 0,054 | 0,142 | 0,307 |
| 40 | SEN BE 1/50 (22/02/91) | 0,058 | 0,057 | 0,143 | 0,082 |
| 41 | SIL RE 1/50 (25/08/00) | 0,056 | 0,057 | 0,131 | 0,107 |
| 42 | SIMO FR 1/50 (29/06/00) | 0,061 | 0,055 | 0,221 | 0,077 |
| 43 | SLI ZB 1/50 (13/11/00) | 0,065 | 0,055 | 0,260 | 0,095 |
| 44 | SOL EM 1/50 (27/11/00) | 0,056 | 0,059 | 0,180 | 0,143 |
| 45 | TAR AL 1/50 (24/07/91) | 0,059 | 0,058 | 0,203 | 0,414 |
| 46 | TEM ST 1/50 (10/05/93) | 0,059 | 0,055 | 0,219 | 0,338 |
| | TEM ST 1/50 (18/11/99) | 0,056 | 0,060 | 0,104 | 0,103 |
| 47 | TER YV 1/50 (02/06/99) | 0,061 | 0,057 | 0,123 | 0,101 |
| | TER YV 1/50 (28/10/99) | 0,104 | 0,076 | 0,122 | 0,412 |
| 48 | THI SY 1/50 (05/06/00) | 0,056 | 0,062 | 0,096 | 0,089 |
| 49 | THO EL 1/50 (04/12/00) | 0,077 | 0,066 | 0,115 | 1,189 |
| 50 | THO MI 1/50 (08/06/00) | 0,057 | 0,057 | 0,146 | 0,078 |
| 51 | THO PA 1/50 (04/12/00) | 0,075 | 0,073 | 0,088 | 0,080 |
| 52 | TOC RO 1/50 (23/11/92) | 0,058 | 0,056 | 0,095 | 0,097 |
| 53 | TOC UR 1/50 (23/11/92) | 0,064 | 0,057 | 0,106 | 0,136 |
| 54 | VAL MAR 1/50 (12/02/92) | 0,060 | 0,058 | 0,098 | 0,084 |
| 55 | VIA JE 1/50 (01/07/93) | 0,098 | 0,077 | 1,426 | 0,099 |
| | VIA JE 1/50 (23/03/00) | 0,059 | 0,053 | 0,181 | 0,073 |

Ainsi, il apparaît à la lecture de ce tableau qu'aucun des sérums testés n'a d'anticorps anti-R7V de type IgG₁ ou IgG₂. Sur 28 patients différents et R7V positifs, 13 patients sont IgG₃ positif (46,4%), 3 patients sont IgG₃ positif et IgG₄ positif (10,7%) et 12 patients sont IgG₄ positifs (42,8%).
Chez un même patient, on retrouve toujours la même sous-classe d'IgG anti-R7V quelque soit la date de prélèvement, lorsque la réponse est positive (BER YV, COU DI, ETC MA, MART DO, SAU CH).

La sous-classe d'anticorps dirigés contre la glycoprotéine d'enveloppe du virus (gp 160) a été déterminée par un tests ELISA (tableau 3).

**Tableau 3 : Détermination de la sous-classe en IgG anti-gp 160 (Kit Sanofi Diagnostics Pasteur)**

| Seuil de positivité = 0,09 | | | | |
|---|---|---|---|---|
| | **IgG**_{**1**} | **IgG**_{**2**} | **IgG**_{**3**} | **IgG**_{**4**} |
| Blanc | 0,065 | 0,062 | 0,060 | 0,067 |
| Témoin séronégatif 1/5 | 0,058 | 0,059 | 0,066 | 0,069 |
| ARG CH 1/5 (30/11/00) | 0,355 | 0,061 | 0,328 | 0,076 |
| AUR PH 1/5 (03/01/94) | 1,645 | 0,068 | 0,160 | 0,781 |
| BER YV 1/5 (25/11/99) | 1,322 | 0,073 | 0,371 | 0,078 |
| CAM GE 1/5 (27/11/00) | 0,121 | 0,050 | 0,066 | 0,051 |
| COU DI 1/5 (16/09/98) | 1,659 | 0,064 | 0,479 | 0,162 |
| DAN NO 1/5 (28/09/00) | 0,855 | 0,058 | 0,361 | 0,069 |
| DAR DO 1/5 (31/08/98) | 1,102 | 0,064 | 0,107 | 0,101 |
| ESM GU 1/5 (18/12/00) | 0,435 | 0,065 | 0,084 | 0,111 |
| ETC MA 1/5 (13/06/00) | 0,513 | 0,060 | 0,135 | 0,172 |
| FIN RO 1/5 (13/11/00) | 0,735 | 0,063 | 1,538 | 0,082 |
| GEM SA 1/5 (07/06/00) | 0,540 | 0,058 | 0,104 | 0,081 |
| MEN CH 1/5 (09/03/00) | 1,260 | 0;054 | 0,209 | 0,069 |
| PAT MA 1/5 (09/07/98) | 0,828 | 0,061 | 0,168 | 0,125 |
| REF EL 1/5 (28/09/00) | 1,511 | 0,073 | 0,252 | 0,080 |
| ROB IS 1/5 (26/06/00) | 0,182 | 0,049 | 0,076 | 0,052 |
| ROD CH 1/5 (12/04/99) | 0,952 | 0,059 | 0,154 | 0,071 |
| SAN FR 1/5 (19/06/00) | 1,112 | 0,063 | 0,157 | 0,076 |
| SAU CH 1/5 (27/11/00) | 0,122 | 0,06 | 0,062 | 1,297 |
| SOL EM 1/5 (27/11/00) | 0,191 | 0,056 | 0,106 | 0,062 |
| TEM ST 1/5 (18/11/99) | 0,834 | 0,061 | 0,069 | 0,082 |
| TER YV 1/5 (28/10/99) | 1,389 | 0,061 | 0,252 | 0,231 |
| THI SY 1/5 (05/06/00) | 0,633 | 0,063 | 0,088 | 0,088 |
| THO EL 1/5 (04/12/00) | 0,899 | 0,057 | 0,158 | 0,064 |
| THO MI 1/5 (08/06/00) | 0,151 | 0,053 | 0,082 | 0,059 |
| THO PA 1/5 (04/12/00) | 0,435 | 0,066 | 0,071 | 0,097 |
| TOC RO 1/5 (25/11/99) | 1,042 | 0,06 | 0,173 | 2,387 |
| VIA JE 1/5 (01/07/93) | 1,748 | 0,080 | 3,062 | 0,127 |

A la lecture de ce tableau, il apparaît qu'aucun des sérums testés n'a d'anticorps anti-gp160 de type IgG₂. Sur 27 patients différents, 27 patients sont IgG₁ positif (100%), 6 patients sont IgG₁ positif uniquement (22,2%), 10 patients sont IgG₁ positif et IgG₃ positif (40,7%), 2 patients sont IgG₁ positif et IgG₄ positif (7,4%), et 9 patients sont IgG₁ positif, IgG₃ positif et IgG₄ (29,6%).

Cette expérience confirme la dispersion de la réponse en anticorps dirigés contre la glycoprotéine d'enveloppe gp160 du virion, et la constance des IgG₃ ou IgG₄ chez les personnes protégées.

### EXEMPLE 2 : Immunoprécipitation du VIH par les IgG₃ totales, purifiées à partir des patients HIV séropositifs et non-progresseurs.

### 2.1. Protocole expérimental:

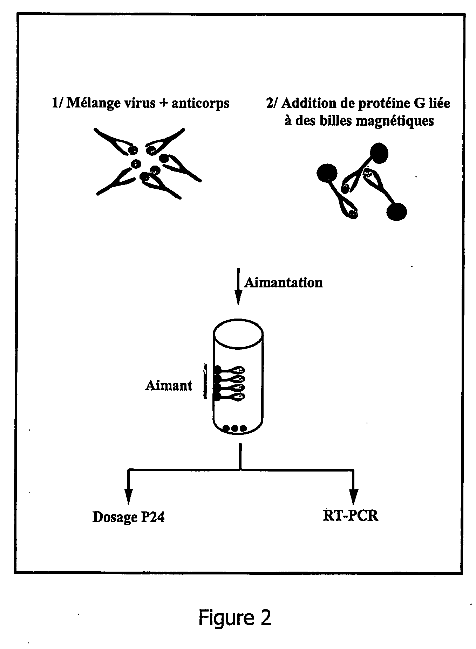

À une quantité donnée d'IgG₃, on ajoute une quantité voulue de virus HIV pur, et le mélange est laissé à incuber 1 heure à +4°C.
On ajoute alors :
- soit 50-100 µl de protéine G fixé sur des billes magnétiques et on incube 1 heure à +4°C (la protéine G fixe les anticorps IgG₁, IgG₂ IgG₃ et IgG₄)
- soit 50-100 µl de protéine A fixé sur des billes magnétiques et on incube 1 heure à +4°C (la protéine A fixe les anticorps IgG₁, IgG₂ et IgG₄ mais pas les IgG₃).
Les billes se fixe à la paroi du tube la plus proche du champ magnétique que l'on applique et le complexe billes de protéine G-IgG₃-virus peut alors être isolé. Le virus lié aux anticorps est ensuite mis en évidence par la mesure de la protéine virale P24 libérée après lyse du virus du virus immunoprécipité (kit COULTER (6604535) et/ou par la technique de RT-PCR pour déceler l'ARN viral. (voir **figure N°2**).

### 2.2. Résultats

Une expérience type est montrée sur le tableau 4 où 65% du virus est précipité par 20µg d'IgG₃ d'un patient HIV positif non-progresseur, alors que les IgG₃ d'un séronégatif sont sans effet.

**Tableau 4 : Immunoprécipitation (virus NDK/PBL du 29/02/96)**

| Echantillon | % Précipitation |
|---|---|
| 20 µg IgG3 ARG CH + 50 µl virus pur + 100 µl protéine G | **64,70%** |
| 10 µg IgG3 ARG CH + 50 µl virus pur + 50 µl protéine G | **56,40%** |
| 20 µg IgG3 Séroneg + 50 µl virus pur + 100 µl protéine G | **0,40%** |
| 10 µg IgG3 Séroneg + 50 µl virus pur + 50 µl protéine G | **0,20%** |
| Témoin virus: 50 µl virus pur + 50 µl protéine G | **0,10%** |
| 20 µg IgG3 ARG CH + 50 µl virus pur + 100 µl protéine A | **0,70%** |
| 10 µg IgG3 ARG CH + 50 µl virus pur + 50 µl protéine A | **0,70%** |
| 20 µg IgG3 Séroneg + 50 µl virus pur + 100 µl protéine A | **0,20%** |
| 10 µg IgG3 Séroneg + 50 µl virus pur + 50 µl protéine A | **0,40%** |
| Témoin virus: 50 µl virus pur + 50 µl protéine A | **0,40%** |

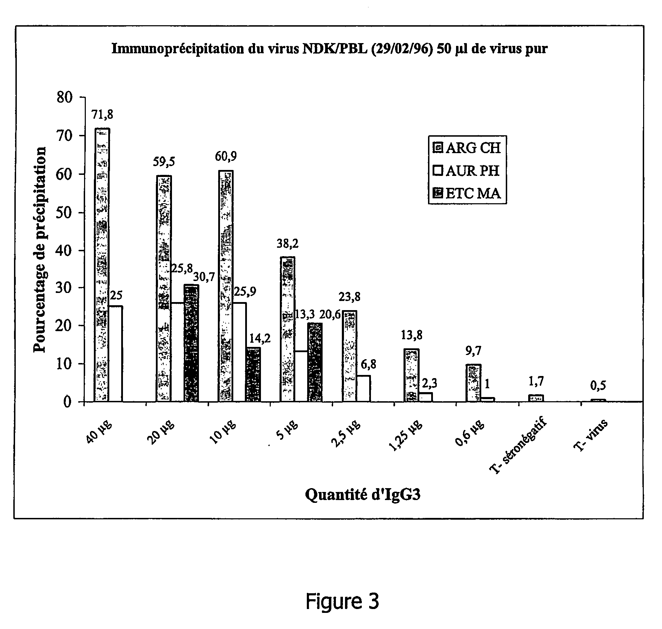

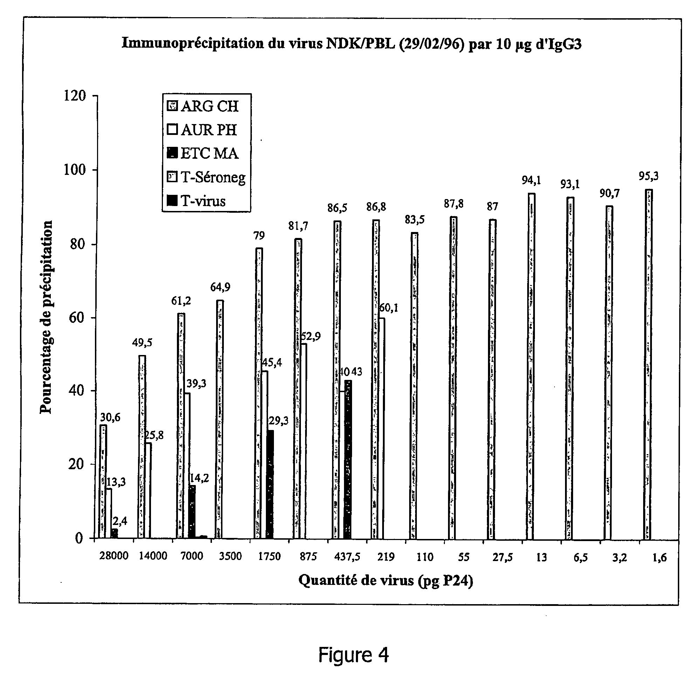

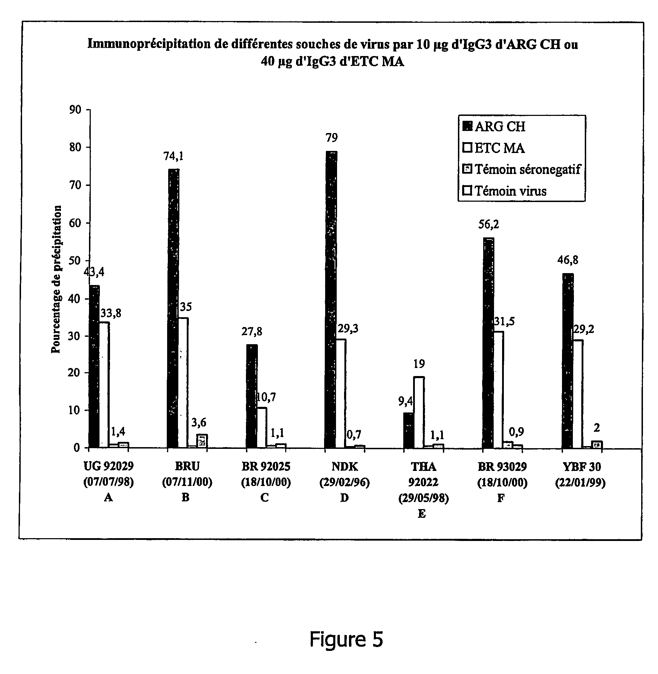

La **figure 3** montre l'immunoprécipitation du virus par les IgG₃ de trois patients différents. Cette immunoprécipitation du virus pour une quantité fixe de virus est dose-dépendante. La **figure 4** montre qu'avec une quantité fixe d'IgG₃, on peut piéger diverses dilutions de virus. La **figure 5** nous indique que quelque soit le type de VIH, il est immunoprécipite par les IgG₃, ici de trois patients. Les souches virales utilisées sont des isolats primaires avec comme phénotype les clades de A à F ou une souche ni O ni M la YBF 30.

Ces expériences confirment que l'on peut isoler un virus par immunoprécipitation à partir des IgG₃ purifiés par la protéine G.

### EXEMPLE 3 : Neutralisation du VIH par les IgG₃ totales, purifiées à partir des patients HIV séropositifs et non-progresseurs.

### 3.1. Protocole expérimental :

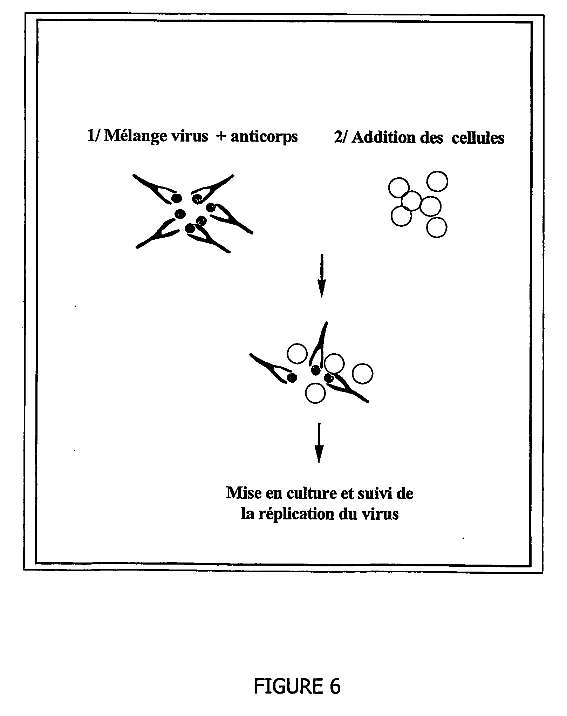

50 µl de solution d'IgG₃ + 50 µl de virus dilué (dans du milieu RPMI 10 %) sont mis à incuber 1 heure à 37°C dans une plaque de 96 puits. Puis 0,3 10⁶ cellules par puits sont ajoutées au mélange et placées 1 heure à 37 °C. Après 2 lavages par du milieu RPMI 0%, les cellules sont mises en plaque de 24 puits dans du milieu RPMI 10% en présence d'IgG₃ (**figure 6**).

### 3.2. Résultats (cf. tableau 5)

**Tableau 5 : Neutralisation de NDK/PBL (29/02/96) par les IgG₃ ou IgG_{1,2,4} d'ARG CH, d'ETC MA, d'AUR PH, et de THO MI.**

| | Dose (µg/ml) | Jour 3 | Jour 4 | Jour 5 | Jour 6⁻ |
|---|---|---|---|---|---|
| **IgG**_{**3**} **ARG CH** | 40 µg/ml | - | (+) | ++ | ++T |
| | | - | (+) | ++ | ++T |
| | 20 µg/ml | - | - | (+) | + |
| | | - | - | + | ++T |
| | 10 µg/ml | (+) | + | ++ | ++T |
| | | (+) | + | ++ | ++T |
| | 5 µg/ml | (+) | - | (+) | + |
| | | (+) | (+) | ++ | ++T |
| **IgG**_{**1,2,4**} **ARG CH** | 40 µg/ml | - | (+) | + | ++T |
| | | - | (+) | ++ | ++T |
| | 20 µg/ml | (+) | + | ++ | ++T |
| | | - | (+) | ++ | ++T |
| | 10 µg/ml | + | ++ | ++ | ++T |
| | | - | (+) | + | ++T |
| | 5 µg/ml | (+) | + | ++ | ++T |
| | | (+) | + | ++ | ++T |
| **IgG**_{**3**} **ETC MA (13/09/01)** | 100 µg/ml | (+) | + | ++ | ++T |
| | | - | (+) | ++ | ++T |
| | 50 µg/ml | (+) | + | ++ | ++T |
| | | - | (+) | ++ | ++T |
| | 25 µg/ml | (+) | ++ | + | ++T |
| | | (+) | ++ | + | ++T |
| | 12,5 µg/ml 12,5 µg/ml | - | - | ++ | ++T |
| | | (+) | (+) | ++ | ++T |
| **IgG**_{**1,2,4**} **ETC MA (13/09/01)** | 100 µg/ml | (+) | ++ | ++ | ++T |
| | | (+) | + | ++ | ++T |
| | 50 µg/ml | (+) | ++ | ++ | ++T |
| | | (+) | + | + | ++T |
| | 25 µg/ml | (+) | (+) | ++ | ++T |
| | | - | - | ++ | ++T |
| | 12,5 µg/ml | (+) | ++ | ++T | ++T |
| | | - | (+) | ++ | ++T |
| **Sérum témoin positif de neutralisation** | 1/50 | - | - | - | - |
| | | - | - | - | - |
| | 1/100 | - | - | - | - |
| | | - | - | - | - |
| Cellules MT4 TEMOIN | | - | - | - | - |
| | | - | - | - | - |
| **VIRUS NDK/PBL (29/02/96)** | **10**^{**-4**} | (+) | + | ++ | ++T |
| | | (+) | + | ++ | ++T |

| | | | | | |
|---|---|---|---|---|---|
| -: absence de syncitia + : présence de syncitia | | | | | |

Les IgG₃ isolés de deux patients neutralisent le VIH comme il est montré dans le tableau 5. À 40 µg et 20 µg d'IgG₃, on constate une neutralisation du virus, c'est à dire une absence de syncitia, alors que les IgG₁, ₂, ₄ sont limités à la dose de 40 µg/ml.

### EXEMPLE 4 : Virolyse du VIH par le complément et les IgG₃ totales, purifiées à partir des patients HIV séropositifs et non-progresseurs.

### 4.1. Protocole expérimentai :

10 ou 20 µg d'IgG3 + 12,5 µl de virus pur sont mis à incuber 1 heure à 4°C sous agitation rotative. Puis 1 ml de complément de lapin au 1/12 (dilution du complément dans du milieu RPMI 10% chauffé à 37°C) est ajouté au mélange, et mis à incuber 30 min à 37°C. Les échantillons sont dilués et la P24 est dosée (kit COULTER 6604535).

### 4.2. Résultats : (tableau 6)

**Tableau 6 : Essai de lyse par le complément (virus NDK/PBL du 29/02/96)**

| Echantillon | % Lyse |
|---|---|
| 20 µg IgG3 ARG CH + 12,5 µl virus + 1 ml complément au 1/12 | **57,60%** |
| 10 µg IgG3 ARG CH + 12,5 µl virus + 1 ml complément au 1/12 | **60,30%** |
| 12,5 µl virus + 1 ml de RPMI 10%, pas de triton | **18,60%** |
| 12,5 µl virus + 1 ml complément au 1/12 | **1,70%** |
| 12,5 µl virus + 1 ml de RPMI 10% + triton | **100%** |

Le complément se fixe sur le complexe IgG₃ - virus et lyse le virus, comme il est montré sur le tableau 6 où 60 % du virus est lysé en présence de 10 µg d'IgG₃.

La neutralisation des virus, et la virolyse en :présence de complément font des IgG₃ un potentiel important pour leur emploi comme anticorps thérapeutiques dans l'infection à VIH ou dans toute autre infection virale où les IgG₃ seront présentes soit de façon classique, soit sous la forme de la variante.

Cette étude suggère que le complément en adjonction avec les anticorps anti-R7V dans le sang des personnes infectées par le VIH, peuvent lyser le virus et détruire son infectivité.
La présence de virus infectieux dans le plasma est de 10 à 100 fois supérieur chez les progresseurs que chez les non-progresseurs. En effet, les patients progresseurs n'ayant pas d'anticorps anti-R7V donc pas d'IgG₃ anti-R7V, il n'y aura pas de neutralisation ni de virolyse, et donc présence de virus.

En conclusion, les inventeurs ont démontré que les anticorps à visée thérapeutique de nature IgG₃ fixant le complément et assurant une virolyse seront très efficaces, non seulement en précipitant et en neutralisant le virus mais encore en le détruisant.

### REFERENCES BIBLIOGRAPHIQUES

Coligan et al., Current Protocols in Immunology [mise à jour annuellement] (4volumes) édités par le "National Institutes of Health" par - Wiley Interscience.
Harlow, et al., (1988) Antibodies: A Laboratory Manual, Cold Spring Harbor Publications pp. 726.
Köhler et Milstein. (1975) Nature, **256**: 495-497.
Sambrook, J., Frischt, E.F. and Maniatis, T. Molecular cloning. A laboratory manual. Cold Spring Harbor Laboratory Press, Cold Spring Harbor, N.Y., 1989.

## Revendications

1. Immunoglobuline humaine de classe IgG3 purifiée et/ou isolée, ayant les caractéristiques suivantes :
- une durée de vie dans le sérum du patient d'au moins un mois ;
- une chaîne lourde dont le poids moléculaire, déterminé par mobilité électrophorétique, est inférieur au poids moléculaire des IgG3 normalement présentes dans le sérum humain qui est 60kDa, la dite chaîne lourde ayant un poids moléculaire d'environ 50 kDa et comprenant le site de fixation du complément.

2. Immunoglobuline selon la revendication 1 **caractérisée en ce que** la concentration de la dite immunoglobuline dans le sérum est supérieure à la concentration d'IgG3 d'un sérum normal d'au moins trois fois, soit environ 1g/l.

3. Immunoglobuline selon les revendications 1 à 2 isolée et/ou purifiée à partir d'un patient affecté d'une pathologie choisie parmi les infections par les virus, les maladies infectieuses à évolution variable telles les maladies neurologiques, les maladies cutanées, les maladies auto-immunes, les cancers d'origine rétrovirale.

4. Immunoglobuline selon la revendication 3, **caractérisée en ce que** le dit virus est sélectionné parmi le virus de l'immunodéficience humaine (HIV), les virus de la leucémie des cellules T humaines (HTLV), le cytomégalovirus (CMV), les virus de l'herpès (HSV-1, HSV-2), le virus d'Epstein-Barr (EBV), les virus de l'hépatite (HBV, HCV).

5. Immunoglobuline selon la revendication 4 **caractérisée en ce que** le dit virus est le HIV et le dit individu est un patient séropositif non-progresseur.

6. Immunoglobuline selon la revendication 5 liant sélectivement l'épitope R7V de la protéine gp160 du virus HIV-1, le dit épitope comprenant la séquence Arg-Thr-Pro-Lys-Ile-Gln-Val.

7. Immunoglobuline selon les revendications 1 à 6, **caractérisée en ce que** la dite immunoglobuline est marquée directement ou indirectement par un marqueur sélectionné parmi les isotopes radioactifs, les enzymes, les colorants, les haptènes, les agents luminescents tels que les agents radioluminescents, chémiluminescents, bioluminescents, fluorescents, phosphorescents, les ligands tels que la biotine, l'avidine, la streptavidine, la digoxygénine.

8. Immunoglobuline selon la revendication 7 **caractérisée en ce que** elle est couplée à un support solide directement ou indirectement par l'intermédiaire d'un bras d'espacement.

9. Procédé de diagnostic et/ou de dosage *in vitro* d'une pathologie sélectionnée parmi les infections virales et les maladies infectieuses à évolution variable, comprenant les étapes de:
- (i) Mise en contact d'un échantillon de fluide corporel et/ou de tissu corporel provenant d'un patient chez lequel on suspecte la présence de la dite pathologie avec une immunoglobuline selon l'une quelconque des revendications 1 à 8; et
- (ii) Détection de la présence d'un complexe immun entre la dite immunoglobuline et un épitope spécifique de là dite pathologie.

10. Procédé de diagnostic et/ou de dosage *in vitro* du syndrome d'immunodéficience humaine (SIDA) comprenant les étapes de:
- (i) Mise en contact d'un échantillon de fluide corporel et/ou de tissu corporel
- provenant d'un patient chez lequel on suspecte la présence du HIV avec une immunoglobuline selon l'une quelconque des revendications 4 à 6,
- (ii) Détection de la présence d'un complexe immun entre la dite immunoglobuline et l'épitope R7V de la protéine gp160 du HIV contenu dans le dit échantillon.

11. Procédé *in vitro* de traitement de fluide biologique d'un patient affecté d'une pathologie sélectionnée parmi les infections virales et les maladies infectieuses à évolution variable, le dit procédé comprenant l'étape de mettre en contact une quantité efficace d'immunoglobuline selon les revendications 1 à 8 avec le dit fluide biologique, de sorte à neutraliser un épitope spécifique de la pathologie dans le dit fluide.

12. Procédé *in vitro* de neutralisation de cellules infectées par le HIV dans une échantillon biologique de fluide corporel et/ou de tissu corporel provenant d'un patient séropositif **caractérisée en ce qu'**il comprend les étapes suivantes:
a) Combiner de manière simultanée, séparée ou étalée dans le temps :
- l'immunoglobuline selon les revendications 5 à 6,
- l'échantillon biologique contenant les cellules infectées par le HIV présentant la protéine gp160 à leur surface, et
- la protéine G attachée à des particules magnétiques ;
b) Incuber le mélange obtenu en a) dans des conditions permettant la fixation de la dite immunoglobuline sur la protéine gp160, et de préférence sur l'épitope R7V, pour former un complexe, le dit complexe comprenant le dit anticorps lié à une cellule infectée par le HIV fixé sur cette particule magnétique ;
c) Déplacer cette particule magnétique vers un endroit prédéterminé du récipient contenant ce mélange réactionnel, tel que le déplacement est accompli par un champ magnétique agissant sur la dite particule magnétique.

13. Procédé *in vitro* de diagnostic de patient non-progresseur affecté d'une pathologie sélectionnée parmi les infections virales et les maladies infectieuses à évolution variable **caractérisé en ce que** l'on détecte la présence d'immunoglobuline IgG3 ayant les caractéristiques suivantes :
- une durée de vie dans le sérum du patient d'au moins un mois ;
- une chaîne lourde dont le poids moléculaire, déterminé par mobilité électrophorétique, est inférieur au poids moléculaire des IgG3 normalement présentes dans le sérum humain (60kDa), la dite chaîne lourde ayant un poids moléculaire d'environ 50 kDa et comprenant le site de fixation du complément.

14. Procédé selon les revendications 11 et 13 **caractérisé en ce que** la dite infection virale est le SIDA.

15. Procédé selon les revendications 13 et 14 **caractérisé en ce que** la détection est réalisée au moyen d'un test immunologique choisi parmi un test ELISA, RIA, une méthode sandwich, une immuno-précipitation, un test d'agglutination.

16. Utilisation d'une immunoglobuline selon les revendications 1 à 8 pour la réalisation d'un test immunologique choisi parmi le test ELISA, le test RIA, la méthode sandwich, l'immuno-précipitation, le test d'agglutination.

17. Immunoglobuline selon la revendication 1 à 7 à titre de médicament.

18. Utilisation d'une immunoglobuline selon la revendication 17 à titre de médicament comme anticorps thérapeutique.

19. Utilisation d'une immunoglobuline selon la revendication 17 à titre de médicament comme agent de ciblage.

20. Utilisation d'une immunoglobuline selon les revendications 1 à 7 pour la préparation d'un médicament destiné au traitement thérapeutique et/ou prophylactique d'une maladie virale ou infectieuse à évolution variable.

21. Utilisation d'une immunoglobuline selon les revendications 1 à 7 pour la préparation d'un médicament destiné au traitement thérapeutique et/ou prophylactique du SIDA.

22. Utilisation selon la revendication 21 d'une immunoglobuline selon les revendications 5 ou 6 pour la préparation d'un médicament destiné à neutraliser chez un homme affecté du SIDA la protéine gp160 de HIV.

23. Vaccin ou composition pharmaceutique pour le traitement ou la prévention d'une pathologie sélectionnée parmi les infections virales et les maladies infectieuses à évolution variable comprenant une immunoglobuline selon les revendications 1 à 7 en association avec un transporteur, un diluant ou un excipient pharmaceutiquement acceptable.

24. Utilisation d'une immunoglobuline selon les revendications 1 à 7 comme marqueur de protection dans les pathologies sélectionnées parmi les maladies virales et les maladies infectieuses à évolution variable.

## Claims

1. Purified and/or isolated human immunoglobulin class IgG3, which has the following characteristics: 5
- a lifespan in the patient's sérum of at least one month;
a heavy chain whose molecular weight, determined by electrophoretic mobility, is less than the molecular weight of the IgG3's normally présent in human sérum, which is 60 kDa, the said heavy chain having a molecular weight of about 50 kDa and comprising the complément binding site.

2. Immunoglobulin according to Claim 1, **charactêrized in that** the concentration of the said immunoglobulin in the sérum is at least three times as high as the IgG3 concentration of a normal sérum, that is about 1 g/1.

3. Immunoglobulin according to either of Claims 1 and 2, isolated and/or purified from a patient affected by a pathology chosen from viral infections, infectious diseases with variable progression such as neurological diseases, skin diseases, autoimmune diseases and cancers of retroviral origin.

4. Immunoglobulin according to Claim 3, **characterized in that** the said virus is selected from the human immunodeficiency virus (HIV), the human T cell leukaemia virus (HTLV), the cytomegalovirus (CMV), the herpesvirus (HSV-1, HSV-2), the Epstein-Barr virus (EBV) and the hepatitis virus (HBV, HCV).

5. Immunoglobulin according to Claim 4, **characterized in that** the said virus is HIV and the said individual is a non-progressor séropositive patient.

6. Immunoglobulin according to Claim 5, selectively binding the R7V epitope of the HIV-1 virus gp160 protein, the said epitope comprising the séquence Arg-Thr-Pro-Lys-Ile-Gln-Val.

7. Immunoglobulin according to Claims 1 to 6, **characterized in that** the said immunoglobulin is directly or indirectly labelled with a marker selected from radioactive isotopes, enzymes, dyes, haptens, luminescent agents such as radioluminescent, chemiluminescent, bioluminescent, fluorescent and phosphorescent agents, ligands such as biotin, avidin, streptavidin and digoxigenin.

8. Immunoglobulin according to Claim 7, **characterized in that** it is directly or indirectly coupled to a solid support via a spacer arm.

9. Method for the in *vitro* diagnosis and/or assay of a pathology selected from viral infections and infectious diseases with variable progression, comprising the steps of:
- (i) bringing a sample of body fluid and/or body tissue obtained from a patient in whom the présence of the said pathology is suspected, into contact with an immunoglobulin according to any one of Claims 1 to 8, and
(ii) detecting the présence of an immune complex between the said immunoglobulin and an epitope spécifie to the said pathology.

10. Method for the *in vitro* diagnosis and/or assay of the human immunodeficiency syndrome (AIDS) comprising the steps of:
(i) bringing a sample of body fluid and/or body tissue obtained from a patient in whom the présence of HIV is suspected, into contact with an immunoglobulin according to any one of Claims 4 to 6,
(ii) detecting the présence of an immune complex between the said immunoglobulin and the R7V epitope of the gp160 protein of the HIV contained in the said sample.

11. *In vitro* method for treating biological fluid from a patient affected by a pathology selected from viral infections and infectious diseases with variable progression, the said method comprising the step of bringing an effective quantity of immunoglobulin according to Claims 1 to 8 into contact with the said 20 biological fluid, so as to neutralize an epitope spécifie to the pathology in the said fluid.

12. *In vitro* method for neutralizing cells infected with HIV in a biological sample of body fluid and/or body tissue obtained from a séropositive patient, **characterized in that** it comprises the following steps:
a) combining simultaneously, separately or spread out over time:
- the immunoglobulin according to Claims 5 to 6,
- the biological sample containing the HIV-infected cells exhibiting the gp160 protein at their surface, and
- the G protein attached to magnetic particles;
b) incubating the mixture obtained in a) under conditions allowing the attachment of the said immunoglobulin to the gp160 protein, and preferably to the R7V epitope, to form a complex, the said complex comprising the said antibody bound to a cell infected with the HIV attached to this magnetic particle;
c) moving this magnetic particle to a predetermined location of the vessel containing this reaction mixture, such that the movement is achieved by a magnetic field acting on the said magnetic particle.

13. *In vitro* method for the diagnosis of a non-10 progressor patient affected by a pathology selected from viral infections and infectious diseases with variable progression, **characterized in that** the présence of immunoglobulin IgG3 is detected, which has the following characteristics:
- a lifespan in the patient's serum of at least one month;
a heavy chain whose molecular weight, determined by electrophoretic mobility, is less than the molecular weight of the IgG3's normally présent in human sérum (60 kDa), the said heavy chain having a molecular weight of about 50 kDa and comprising the complément binding site.

14. Method according to Claims 11 and 13, **characterized in that** the said viral infection is AIDS.

15. Method according to Claims 13 and 14, **characterized in that** the détection is carried out by means of an immunological test chosen from an ELISA test, an RIA test, a sandwich method, an immuno-precipitation and an agglutination test.

16. Use of an immunoglobulin according to Claims 1 to 8, for carrying out an immunological test chosen from the ELISA test, the RIA test, the sandwich method, immunoprecipitation and the agglutination test.

17. Immunoglobulin according to Claims 1 to 7, as a médicament.

18. Use of an immunoglobulin according to Claim 17, as a therapeutic antibody, as medicament.

19. Use of an immunoglobulin according to Claim 17, as a targeting agent, as medicament.

20. Use of an immunoglobulin according to Claims 1 to 7, for the préparation of a médicament intended for the therapeutic and/or prophylactic treatment of a viral disease or an infectious disease with variable progression.

21. Use of an immunoglobulin according to Claims 1 to 7, for the préparation of a médicament intended for the therapeutic and/or prophylactic treatment of AIDS.

22. Use according to Claim 21, of an immunoglobulin according to Claim 5 or 6, for the préparation of a médicament intended to neutralize the HIV gp160 protein in humans affected by AIDS.

23. Vaccine or pharmaceutical composition for the treatment or prévention of a pathology selected from viral infections and infectious diseases with variable progression comprising an immunoglobulin according to Claims 1 to 7, in combination with a pharmaceutically acceptable vehicle, diluent or excipient.

24. Use of an immunoglobulin according to Claims 1 to 7, as a protective marker in pathologies selected from viral diseases and infectious diseases with 35 variable progression.

## Patentansprüche

1. Gereinigtes und/oder isoliertes humanes Immunglobulin der Klasse IgG3, welches die folgenden Merkmale aufweist:
- eine Lebensdauer im Serum des Patienten von wenigstens einem Monat;
- eine schwere Kette, deren Molekulargewicht, bestimmt anhand von elektrophoretischer Mobilität, geringer ist als das Molekulargewicht der normalerweise im menschlichen Serum vorhandenen lgG3, welches 60 kDa beträgt, wobei die schwere Kette ein Molekulargewicht von ungefähr 50 kDa aufweist und die Komplementbindungsstelle umfasst.

2. Immunglobulin nach Anspruch 1, **dadurch gekennzeichnet, dass** die Konzentration des Immunglobulins im Serum wenigstens dreimal höher ist als die Konzentration von IgG3 eines normalen Serums, was ungefähr 1 g/l entspricht.

3. Immunglobulin nach den Ansprüchen 1 bis 2, welches ausgehend von einem von einer Pathologie, ausgewählt unter den Infektionen durch Viren, den Infektionskrankheiten mit variabler Entwicklung, wie den neurologischen Erkrankungen, den Hauterkrankungen, den Autoimmunerkrankungen, den Krebserkrankungen retroviralen Ursprungs, befallenen Patienten isoliert und/oder gereinigt worden ist.

4. Immunglobulin nach Anspruch 3, **dadurch gekennzeichnet, dass** das Virus ausgewählt wird unter dem "human immunodeficiency virus" (HIV), den humanen T-Zell-Leukämieviren (HTLV), dem Zytomegalievirus (CMV), den Herpes-Viren (HSV-1, HSV-2), dem Epstein-Barr-Virus (EBV), den Hepatitis-Viren (HBV, HCV).

5. Immunglobulin nach Anspruch 4, **dadurch gekennzeichnet, dass** das Virus das HIV ist und das Individuum ein seropositiver "Non-Progressor"-Patient ist.

6. Immunglobulin nach Anspruch 5, welches selektiv das Epitop R7V des Proteins gp160 des HIV-1-Virus bindet, wobei das Epitop die Sequenz Arg-Thr-Pro-Lys-Ile-Gln-Val umfasst.

7. Immunglobulin nach den Ansprüchen 1 bis 6, **dadurch gekennzeichnet, dass** das Immunglobulin direkt oder indirekt durch einen Marker, ausgewählt unter den radioaktiven Isotopen, den Enzymen, den Färbemitteln, den Haptenen, den lumineszierenden Mitteln, wie den radiolumineszierenden, chemolumineszierenden, biblumineszierenden, fluoreszierenden, phosphoreszierenden Mitteln, den Liganden, wie Biotin, Avidin, Streptavidin, Digoxigenin, markiert ist.

8. Immunglobulin nach Anspruch 7, **dadurch gekennzeichnet, dass** es direkt oder indirekt über einen Spacer-Arm mit einem festen Träger verknüpft ist.

9. *In vitro*-Verfahren zur Diagnose und/oder zur quantitativen Bestimmung einer Pathologie, ausgewählt unter den Virusinfektionen und den Infektionskrankheiten mit variabler Entwicklung, welches die Schritte umfasst:
- (i) Inkontaktbringen einer Probe von Körperflüssigkeit und/oder von Körpergewebe, welche von einem Patienten stammt, bei welchem man das Vorliegen der Pathologie vermutet, mit einem Immunglobulin nach einem der Ansprüche 1 bis 8; und
- (ii) Detektion des Vorhandenseins eines Immunkomplexes zwischen dem Immunglobulin und einem für die Pathologie spezifischen Epitop.

10. *In vitro*-Verfahren zur Diagnose und/oder zur quantitativen Bestimmung des humanen Immundefektsyndroms (AIDS), welches die Schritte umfasst:
- (i) Inkontaktbringen einer Probe von Körperflüssigkeit und/oder von Körpergewebe, welche von einem Patienten stammt, bei welchem man das Vorhandensein des HIV vermutet, mit einem Immunglobulin nach einem der Ansprüche 4 bis 6;
- (ii) Detektion des Vorhandenseins eines Immunkomplexes zwischen dem Immunglobulin und dem Epitop R7V des Proteins gp160 des HIV, welches in der Probe enthalten ist.

11. *In vitro*-Verfahren zur Behandlung eines biologischen Fluids von einem Patienten, welcher von einer Pathologie, ausgewählt unter den Virusinfektionen und den Infektionskrankheiten mit variabler Entwicklung, befallen ist, wobei das Verfahren den Schritt umfasst, eine wirksame Menge Immunglobulin nach den Ansprüchen 1 bis 8 mit dem biologischen Fluid in Kontakt zu bringen derart, dass ein für die Pathologie spezifisches Epitop in dem Fluid neutralisiert wird.

12. *In vitro*-Verfahren zur Neutralisation von durch das HIV infizierten Zellen in einer biologischen Probe von Körperflüssigkeit und/oder von Körpergewebe, welche von einem seropositiven Patienten stammt, **dadurch gekennzeichnet, dass** es die folgenden Schritte umfasst:
a) gleichzeitig, getrennt oder zeitlich gestaffelt zusammenzugeben:
- das Immunglobulin nach den Ansprüchen 5 bis 6,
- die biologische Probe, welche die durch das HIV infizierten Zellen, die auf ihrer Oberfläche das Protein gp160 aufweisen, enthält, und
- das an magnetische Teilchen angeheftete Protein G;
b) die in a) erhaltene Mischung zu inkubieren unter Bedingungen, welche die Bindung des Immunglobulins an das Protein gp160 und bevorzugt an das Epitop R7V erlauben, um einen Komplex zu bilden, wobei der Komplex den Antikörper gebunden an eine durch das HIV infizierte Zelle, fixiert auf diesem magnetischen Teilchen, umfasst;
c) dieses magnetische Teilchen in Richtung einer vorher festgelegten Stelle des Behälters, welcher diese Reaktionsmischung enthält, zu verschieben, wobei die Verschiebung beispielsweise durch ein magnetisches Feld, welches auf das magnetische Teilchen einwirkt, bewerkstelligt wird.

13. *In vitro*-Verfahren zur Diagnose eines "Non-Progressor"-Patienten, welcher von einer Pathologie, ausgewählt unter den Virusinfektionen und den Infektionskrankheiten mit variabler Entwicklung, befallen ist, **dadurch gekennzeichnet, dass** man das Vorhandensein von Immunglobulin IgG3, welches die folgenden Merkmale aufweist:
- eine Lebensdauer im Serum des Patienten von wenigstens einem Monat;
- eine schwere Kette, deren Molekulargewicht, bestimmt anhand von elektrophoretischer Mobilität, geringer ist als das Molekulargewicht der normalerweise im menschlichen Serum vorhandenen IgG3 (60 kDa), wobei die schwere Kette ein Molekulargewicht von ungefähr 50 kDa aufweist und die Komplementbindungsstelle umfasst,
detektiert.

14. Verfahren nach den Ansprüchen 11 und 13, **dadurch gekennzeichnet, dass** die Virusinfektion AIDS ist.

15. Verfahren nach den Ansprüchen 13 und 14, **dadurch gekennzeichnet, dass** die Detektion mittels eines immunologischen Tests, ausgewählt unter einem ELISA-Test, einem RIA-Test, einem Sandwich-Verfahren, einer Immunpräzipitation, einem Agglutinationstest, ausgeführt wird.

16. Verwendung eines Immunglobulins nach den Ansprüchen 1 bis 8, für die Ausführung eines immunologischen Tests, welcher unter dem ELISA-Test, dem RIA-Test, dem Sandwich-Verfahren, der Immunpräzipitation, dem Agglutinationstest ausgewählt wird.

17. Immunglobulin nach Anspruch 1 bis 7 als Arzneimittel.

18. Verwendung eines Immunglobulins nach Anspruch 17 als Arzneimittel als therapeutischer Antikörper.

19. Verwendung eines Immunglobulins nach Anspruch 17 als Arzneimittel als Targeting-Mittel.

20. Verwendung eines Immunglobulins nach den Ansprüchen 1 bis 7 für die Herstellung eines Arzneimittels, welches für die therapeutische und/oder prophylaktische Behandlung einer Viruserkrankung oder Infektionskrankheit mit variabler Entwicklung bestimmt ist.

21. Verwendung eines Immunglobulins nach den Ansprüchen 1 bis 7 für die Herstellung eines Arzneimittels, welches für die therapeutische und/oder prophylaktische Behandlung von AIDS bestimmt ist.

22. Verwendung nach Anspruch 21 eines Immunglobulins nach den Ansprüchen 5 oder 6 für die Herstellung eines Arzneimittels, welches dafür bestimmt ist, bei einem von AIDS befallenen Menschen das Protein gp160 von HIV zu neutralisieren.

23. Impfstoff oder pharmazeutische Zusammensetzung für die Behandlung oder die Verhütung einer Pathologie, ausgewählt unter den Virusinfektionen und den Infektionskrankheiten mit variabler Entwicklung, umfassend ein Immunglobulin nach den Ansprüchen 1 bis 7 in Kombination mit einem Transportmittel, einem Verdünnungsmittel oder einem Träger, welche pharmazeutisch annehmbar sind.

24. Verwendung eines Immunglobulins nach den Ansprüchen 1 bis 7 als Schutzmarker bei den Pathologien, welche unter den Viruserkrankungen und den Infektionskrankheiten mit variabler Entwicklung ausgewählt werden.
